# EUROPÄISCHE PATENTANMELDUNG

(11) **EP 1 857 123 A1**
(43) Veröffentlichungstag der Anmeldung: **21.11.2007**
(21) Anmeldenummer: 07009015.4
(22) Anmeldetag: 04.05.2007
(51) Int. Cl.: A61L 2/04, A61L 2/07, A61L 2/08, A61L 2/20, A61K 6/10, A61K 6/093

(54) **Verfahren zur Sterilisation von Abformmaterialien und sterilisierbares Abformmaterial**

(30) Priorität: 15.05.2006 DE 102006022880
(71) Anmelder: Kettenbach GmbH & CO. KG, 35713 Eschenburg (DE)
(72) Erfinder: Bublewitz, Alexander, Dr., 35745 Herborn (DE); Reber, Jens-Peter, Dr., 58540 Meinerzhagen (DE); Suchan, Matthias, Dr., 57627 Hachenburg (DE)
(74) Vertreter: KEIL & SCHAAFHAUSEN Patentanwälte

(57) **Zusammenfassung**

Die Erfindung betrifft ein Verfahren zur Sterilisation von aushärtbaren, medizinischen Ein- oder Mehrkomponenten-Abformmaterialien, wobei in einem ersten Schritt die nicht ausgehärteten Komponenten der Abformmaterialien jeweils in eine Primärverpackung (1, 6, 8) eingebracht werden, in einem zweiten Schritt die Primärverpackungen mit den darin enthaltenen Komponenten durch eine Hitzesterilisation sterilisiert werden, in einem dritten Schritt die sterilisierten Komponenten in den Primärverpackungen in eine Sekundärverpackung (5, 7, 9) eingebracht werden und in einem vierten Schritt diese Sekundärverpackung durch eine geeignete Gas-, Strahlen- und/oder Sterilisation in einem Autoklaven derart sterilisiert wird, dass sich die Aktivität und die Viskosität der Einzelkomponenten nicht ändert. Weiter betrifft die Erfindung ein Zwei-Komponenten-Silicon-Abformmaterial.

## Beschreibung

Die vorliegende Erfindung betrifft ein insbesondere mehrstufiges Verfahren zur Sterilisation von aushärtbaren, medizinischen Ein- oder Mehrkomponenten-Abformmaterialien. Weiter betrifft die Erfindung ein sterilisierbares Abformmaterial.

Abformmaterialien finden in großer Zahl und in verschiedensten Ausführungsformen, sei es was die Anzahl der Komponenten angeht, sei es was die Zusammensetzungen der einzelnen Komponenten oder die Art der Abbindung betrifft, Anwendung.

So kommen z.B. Systeme zum Einsatz, die durch Additionsreaktion abbinden, wie additionsvernetzende Silicon-Abformmaterialien oder entsprechende Polyether-Abformmaterialien. Alternativ finden kondensationsvernetzende Systeme Anwendung; als Beispiele seien hier kondensationsvernetzende Silicon- und Polyether-Abformmaterialien genannt.

Überwiegend kommen diese Materialien als Zwei-Komponenten-Systeme zum Einsatz, die nach dem Vermischen in mehr oder weniger kurzer Zeit abbinden, d. h. aushärten.

Im für medizinische Zwecke vorgesehenen Einsatzbereich tritt jedoch oftmals das Problem auf, dass das anfangs pastöse Material mit Haut- oder Schleimhautverletzungen oder chirurgisch freigelegten Strukturen im Mund bzw. in der Endoprothetik z. B. bei Hüftgelenk- oder Kniegelenkprothesen in Kontakt kommt, wobei die Gefahr besteht, dass beispielsweise Bakterien, Pilze oder Viren übertragen werden. Dies kann zu erheblichen gesundheitlichen Beeinträchtigungen führen. Darüber hinaus kann der Einsatz eines sterilen aushärtbaren Materials bspw. als Wurzelfüllmaterial in der Zahnmedizin erforderlich sein.

Ähnliche Probleme können auch auftreten, wenn zwar das Material an sich keimfrei gehalten wird, aber die zu dessen Anwendung gebrauchten Gegenstände wie z. B. Abformlöffel, Mischer oder Kartuschenkörper nicht steril und daher keimbeladen sind.

Aus diesem Grund besteht ein starkes Interesse an einem sterilen Abformmaterial und an sterilen Gerätschaften zu dessen Applikation.

Die EP 1 477 151 A1 beschreibt eine Sterilisationsmethode, bei der eine Sterilisation durch Kochen bei 100°C beziehungsweise Autoklavieren bei 121°C stattfindet. Auch wird eine Sterilisation mit Gammastrahlen sowie die Anwendung von Elektronen- und Röntgenstrahlen vorgeschlagen, die allerdings das Abformmaterial schädigen kann.

In der EP 1 374 914 A1 wird ein Verfahren vorgestellt, in dem Abformmaterialien und/oder deren Komponenten einer Dampfsterilisation unterworfen werden. Hierbei kommen als so genannte Primärverpackungen Dosen, Schlauchbeutel, Tuben, Spritzen und Doppelkammerkartuschen zum Einsatz, in denen die Komponenten enthalten sind. Diese werden zusammen mit für die Applikation der Abformmassen benötigtem Zubehör, wie z. B. Mischdüsen, in eine Sterilisierverpackung eingebracht. Dieses Gebinde wird dann einer Dampfsterilisation unterworfen. Dabei beträgt nach der Lehre der EP 1 374 914 A1 die einzustellende Temperatur vorzugsweise höchstens 138°C und die Dauer des Verfahrens höchstens 30 Minuten. Durch längere Reaktionszeiten und höhere Temperaturen kann das Material nach der Lehre der EP 1 374 914 A1 irreparablen Schaden erleiden.

Ein weiterer Nachteil ist, dass bei dieser Methode, d. h. der gleichzeitigen Sterilisation aller Bestandteile, also der zu vernetzenden Massen und der dafür verwendeten Gerätschaften, nur Bedingungen angewandt werden können, die für alle Teile in Bezug auf deren Stabilität tolerierbar sind. Nicht dampfsterilisierbare Teile, wie z. B. Polyethylenverschlusskappen nehmen sonst ebenfalls irreparablen Schaden.

Ein ähnliches Konzept verfolgt die EP 1 374 915 A1. Hier findet der vorgenommene Sterilisationsschritt durch eine Strahlensterilisation statt. Allerdings wirkt sich hierbei nachteilig aus, dass insbesondere additionsvernetzende Silicon-Abformmaterialien bei höheren Strahlendosen schon vorvernetzen und damit für einen anschließenden bestimmungsgemäßen Gebrauch nicht mehr verwendbar sind.

Aufgabe der vorliegenden Erfindung ist es daher, ein Verfahren zur Sterilisation von aushärtbaren, medizinischen Ein- oder Mehrkomponenten-Abformmaterialien zur Verfügung zu stellen, bei dem sowohl das Ein- oder Mehrkomponentenmaterial als auch dessen Umverpackung (Primärverpackung) und gegebenenfalls auch zur Applikation verwendete Gerätschaften zuverlässig sterilisiert werden, ohne dass es zu einer Vorschädigung, insbesondere Vorvernetzung, verminderter Lagerstäbilität, Viskositätsveränderung oder Reaktivitätsveränderung des Ein- oder Mehrkomponentenmaterials oder einer Schädigung der Gerätschaften kommt.

Erfindungsgemäß wird diese Aufgabe durch ein Verfahren mit folgenden Schritten gelöst: Verpacken der Komponenten einzeln oder gemeinsam in wenigstens eine hitzebeständige Verpackung, wobei zumindest eine der Verpackungen gasdicht ist, und anschließendes Sterilisieren der Komponenten durch vorzugsweise trockene Hitze bei einer Temperatur von über 138° C und bei etwa Normaldruck. Die Sterilisation bei der erfindungsgemäß hohen Temperatur von über 138°C hat den Vorteil, dass nicht nur äußere Bereiche einer bspw. in einem Schlauchbeutel oder einer Kartusche aufgenommenen Komponente sondern auch deren Kern ausreichend erwärmt wird, um die Komponente zu sterilisieren. Die gasdichte Verpackung verhindert dabei eine Oxidation der Komponenten mit Umgebungsluft. Für das erfindungsgemäße Verfahren können unterschiedliche Verpackungen eingesetzt werden. So können die Komponenten direkt in einer hitzestabilen und möglichst gasundurchlässigen Kartusche, die bspw. fluoriert ist und mit einer Aluminiumverbundfolie verschlossene Öffnungen aufweist, aufgenommen sein. Alternativ hierzu ist es auch möglich, die Komponenten in einem Schlauchbeutel oder einem anderen geeigneten Beutel aus einer Metallverbundfolie aufzunehmen.

Bei dem erfindungsgemäßen Verfahren können die Komponenten bei der Sterilisation mit trockener Hitze entweder in einer gasdichten Primärverpackung aufgenommen sein, wobei keine oder eine gasdichte oder gasdurchlässige Sekundärverpackung vorgesehen werden kann, oder die Komponenten sind bei der Sterilisation mit trockener Hitze entweder in einer nicht gasdichten Primärverpackung aufgenommen, wobei dann eine gasdichte Sekundärverpackung vorgesehen ist.

Nach einer bevorzugten Ausführungsform des erfindungsgemäßen Verfahrens wird vor dem gasdichten Verschließen der Verpackung Umgebungsluft vorzugsweise nahezu vollständig aus der Verpackung entfernt und/oder die Verpackung mit Inertgas befüllt. Hierdurch wird eine Oxidationsreaktion der Komponenten, die diese unbrauchbar machen könnte, besonders wirkungsvoll vermieden. Dabei kann es ausreichend sein, über einen weichen Stempel, bspw. einen Schaumstoffstempel, die Restluft nur weitgehend aus der Verpackung auszubringen, so dass allenfalls nur noch kleine Luftreste in der Verpackung verbleiben. Es wird jedoch bevorzugt, wenn die Restluft zumindest nahezu vollständig aus der Verpackung abgesaugt und/oder die Verpackung mit Schutzgas befüllt wird.

Eine besonders bevorzugte Ausführungsform der Erfindung sieht vor, dass in einem ersten Schritt die nicht ausgehärteten Komponenten der medizinischen Abformmaterialien jeweils in eine Primärverpackung eingebracht werden, in einem zweiten Schritt die Primärverpackungen mit den darin enthaltenen Komponenten durch eine Hitzesterilisation, insbesondere durch trockene Hitze, sterilisiert werden, in einem dritten Schritt die sterilisierten Komponenten in den Primärverpackungen in eine Sekundärverpackung eingebracht werden und in einem vierten Schritt diese Sekundärverpackung durch eine geeignete, d.h. in Konzentration und Dauer angemessene Gassterilisation und/oder durch eine geeignete Strahlensterilisation, d.h. eine Strahlensterilisation mit einer Strahldosis, die hoch genug ist, die Oberflächen der Primärverpackung und der ggf. zusätzlich vorgesehenen Gerätschaften zur Applikation zu sterilisieren und gleichzeitig so niedrig ist, dass das Abformmaterial nicht geschädigt wird, und/oder durch eine in Dauer, Druck und Temperatur angemessene Sterilisation im Autoklaven derart sterilisiert wird, dass sich die Aktivität und die Viskosität der Komponenten nicht ändert. Im Sinne der vorliegenden Erfindung ist darunter zu verstehen, dass sich die Aktivität der Komponenten so wenig ändert, dass sich die Aushärtezeit nicht um ein Δ von mehr oder weniger als einer Minute verlängert oder verkürzt, bzw. dass die Änderung der Viskosität der Komponenten weniger als etwa 20%, bevorzugt weniger als 10%, ganz besonders bevorzugt weniger als 5% beträgt. Alternativ zu einer Hitzesterilisation durch trockene Hitze kann die Primärverpackung in dem zweiten Schritt auch durch (Wasser-)Dampf oder durch Kochen sterilisiert werden.

Der erste Sterilisationsschritt ist eine Hitzesterilisation bei einer für das Material geeigneten Temperatur. Im Gegensatz zu einer Sterilisation mittels energiereicher Gammastrahlung findet eine Vorschädigung bzw. Vorvernetzung des Abdruckmaterials hierbei nicht statt.

Die Primärverpackungen sind vorzugsweise derart gewählt, dass sie während der Hitzesterilisation keinen Schaden nehmen. Für die Primärverpackung eignen sich bspw. die nachfolgenden hochtemperaturbeständigen Kunststoffe: cycloolefinische Copolymere (z.B. Topas® 6015, Fa. Ticona), (Co-) Polycarbonate (z.B. APEC® 1745, Fa. Bayer) oder Polysulfon (z.B. Ultrason® S 3010, Fa. BASF). Weiter eignen sich besonders Kunststoff-Metall-Kunststoff-Verbundverpackungen, insbesondere Folien- bzw. Blisterverpackungen, Metall- oder Metallverbundtuben, Metallverbundfolienschlauchbeutel, Metall- oder Metallverbundkartuschen, insbesondere Polypropylen-Aluminium-Polypropylen-Verbundverpackungen, Aluminiumtuben, Zinntuben, Aluminium-Zinn-Verbundtuben, Aluminiumkartuschen, Zinnkartuschen oder Aluminium-Zinn-Verbundkartuschen. Dabei können die Metallkartuschen, -tuben, -folien bzw. -blister auch Innenbeschichtungen aufweisen. Die verwendeten Kunststoffprimärverpackungen können auch fluoriert sein. Diese Materialien können höheren Temperaturen ausgesetzt werden als beispielsweise herkömmliche Polyethylen-Umverpackungen.

In einer bevorzugten Ausführungsform wird die Primärverpackung mit einem Aktivierungskopf versehen oder in eine Kartusche eingebracht und mit einem Kolben verschlossen. Derartige Verbindungen einer bspw. als Folienbeutelverpackung ausgebildeten Primärverpackung mit Komponenten eines Systems zum Mischen und/oder Austragen der in der Primärverpackung aufgenommenen Komponenten sind beispielsweise in der EP 1 065 153, der EP 1 169 242, der EP 1 138 397, der EP 1 112 779 sowie in der EP 1 121 195 beschrieben.

Neben den üblichen Hitzesterilisationsverfahren findet bevorzugt die trockene Hitzesterilisation bei einer Temperatur T größer als 138°C und kleiner oder gleich 160°C, bevorzugt bei einer Temperatur T größer oder gleich 140°C und kleiner oder gleich 150°C und besonders bevorzugt bei einer Temperatur T grö-ßer oder gleich 140°C und kleiner oder gleich 145°C, statt.

Dabei wird bei der Hitzesterilisation bevorzugt eine Behandlungszeit von größer oder gleich einer Stunde, besonders bevorzugt eine Zeit von größer oder gleich 1,5 Stunden und ganz besonders bevorzugt eine Zeit von größer als 2 Stunden ab Erreichen der Kerntemperatur gewählt.

Durch diese Wahl der Parameter Temperatur und Behandlungszeit wird gewährleistet, dass die Keimreduzierung in dem behandelten Material optimal ist und das sterilisierte Material für die vorgesehenen medizinischen Zwecke bedenkenlos eingesetzt werden kann.

In einem zweiten Schritt werden die sterilisierten Primärverpackungen in eine Sekundärverpackung eingebracht und dieses Gebinde wird einer zweiten Sterilisation unterworfen. Dies dient der Sterilisation der Außenseite der Primärverpackungen, die bei der weiteren Verarbeitung, z. B. Konfektionierung, kontaminiert werden kann.

Als Sekundärverpackung kann jede bekannte Verpackungsform dienen, die verschließbar ist und den Bedingungen der zweiten Sterilisation Stand hält. In einer besonders bevorzugten Ausführungsform ist die Sekundärverpackung ein Kunststoffbeutel bzw. Sterilbeutel, der transparent, opak und/oder bedruckt sein kann. Vorzugsweise ist die Sekundärverpackung ebenfalls temperaturbeständig. Diese kann bspw. aus einem der nachfolgenden hochtemperaturbeständigen Kunststoffe bestehen: cycloolefinische Copolymere (z.B. Topas® 6015, Fa. Ticona), (Co-) Polycarbonate (z.B. APEC® 1745, Fa. Bayer) oder Polysulfon (z.B. Ultrason® S 3010, Fa. BASF).

Bei der zweiten Sterilisation handelt es sich bspw. um eine geeignete Gas-, Strahlensterilisation und/oder eine Sterilisation im Autoklaven. Bei der Gassterilisation wird bspw. eine Sterilisation mit Ethylenoxid (EO, Oxiran) bevorzugt. Die Strahlensterilisation ist vorzugsweise eine geeignete α-, β-Strahlen- oder Röntgenstrahlensterilisation.

Dabei kann die Behandlungszeit in diesem zweiten Schritt wesentlich kleiner sein als die des ersten Sterilisationsschrittes. Bevorzugt ist hierbei eine Behandlungszeit von kleiner oder gleich einer Stunde, insbesondere kleiner oder gleich 30 Minuten und besonders bevorzugt kleiner oder gleich 10 Minuten. Dadurch wird zum Einen die Möglichkeit von Schädigungen der Materialien, insbesondere der Abformmaterialien unterbunden und zum Zweiten eine akzeptable Gesamtdauer des Sterilisationsprozesses bei gleichzeitig ausreichender Sterilisationsqualität erreicht.

Insgesamt wird durch dieses Verfahren ein steriles Material erhalten, bei dem die Komponenten nicht durch Vorvernetzung unbrauchbar werden und/oder der Katalysator geschädigt wird und das nach der Sterilisation ausreichend lange, z. B. 24 Monate, lagerstabil ist.

Vorzugweise wird in die Sekundärverpackung auch das zur Applikation der Abformmasse benötigte Zubehör, wie z. B. ein dynamischer oder statischer Mischer, Abformlöffel, Aktivierungsköpfe, Kartuschen und/oder Kartuschenkörper, eingebracht. Durch die kürzere und im Hinblick auf die Temperatur schonendere zweite Sterilisation im Vergleich mit einer Hitzesterilisation wie sie im ersten Schritt angewendet wird, nimmt dieses Zubehör keinen Schaden, auch wenn es aus nicht hitzebeständigem Material hergestellt ist.

Nach einer vorteilhaften Ausführungsform der Erfindung werden die Komponenten des Ein- oder Mehrkomponentenabformmaterials in eine entsprechende Anzahl von Schlauchbeuteln gefüllt, welche nach einer Sterilisation durch trockene Hitze mit einem Dichtring und einem Aktivierungskopf bekappt werden. Anschließend werden die Schlauchbeutel mit Dichtring und Aktivierungskopf in einen Sterilbeutel verpackt und versiegelt, der durch eine geeignete Ethylenoxidsterilisation und/oder geeignete α-, β- und/oder Röntgenstrahlensterilisation und/oder eine geeignete Sterilisation im Autoklaven sterilisiert wird. Ebenso können Zubehörteile, wie z. B. dynamische oder statische Mischer und/oder wiederverwendbare Kartuschenkörper einzeln oder gemeinsam in Sterilbeutel verpackt und versiegelt werden und ebenfalls durch eine geeignete Ethylenoxidsterilisation und/oder geeignete α-, β- und/oder Röntgenstrahlensterilisation und/oder geeignete Sterilisation im Autoklaven sterilisiert werden. Hierbei ist es auch möglich, die bekappten sterilisierten Schlauchbeutel und einzelne oder mehrere Zubehörteile in einen gemeinsamen Sterilbeutel zu verpacken, so dass diese gemeinsam einer Ethylenoxidsterilisationen und/oder eine geeignete α-, β- und/oder Röntgenstrahlensterilisationen und/oder Sterilisationen im Autoklaven unterworfen werden.

Nach einer weiteren Ausführungsform der Erfindung werden die durch trockene Hitze in Schlauchbeuteln, die einen Inhalt von etwa 1 ml bis bspw. etwa 400 ml aufweisen können, sterilisierten Komponenten in eine geeignete Kartusche eingebracht und in dieser verschlossen. Die Kartuschen, die vorzugsweise als Doppelkartuschen zwei Schlauchbeutel aufnehmen, werden dann einzeln oder zusammen mit Zubehörteilen, wie z. B. einem oder mehreren statischen Mischern und/oder einer Austragspistole, in Sterilbeutel verpackt und versiegelt. Je nach Verpackungsart können die Kartuschen und Zubehörteile somit einzeln oder gemeinsam durch eine geeignete Ethylenoxidsterilisation und/oder geeignete α- β- und/oder Röntgenstrahlensterilisation und/oder Sterilisation im Autoklaven sterilisiert werden.

Die Komponenten können nach einer weiteren bevorzugten Ausführungsform der Erfindung in eine für die Sterilisation geeignete, direkt befüllbare Kunststoff-, Metall- oder metallbeschichtete Kartusche, insbesondere eine Doppelkartusche, gefüllt werden, die dann verschlossen und einer Sterilisation durch trockene Hitze unterworfen wird. Die derart sterilisierte Kartusche mit den Komponenten wird anschließend alleine oder zusammen mit Zubehörteilen, wie bspw. einem oder mehreren statischen Mischern und/oder einer Austragspistole, in einen Sterilbeutel verpackt. Somit kann die Kartusche einzeln oder gemeinsam mit Zubehörteilen durch eine geeignete Ethylenoxidsterilisation und/oder geeignete α-, β- und/oder Röntgenstrahlensterilisation und/oder Sterilisation im Autoklaven sterilisiert werden.

Wenn die Komponenten in eine direkt befüllbare Blisterverpackung als Primärverpackung eingefüllt sind, wird diese nach einer weiteren Ausführungsform der Erfindung nach dem Verschließen einer Sterilisation durch trockene Hitze unterworfen. Die sterilisierte Blisterverpackung mit den Komponenten wird zusammen mit Mischzubehör, wie einem Spatel oder dgl., in einen Sterilbeutel verpackt, der durch eine geeignete Ethylenoxidsterilisation und/oder geeignete α-, β- und/oder Röntgenstrahlensterilisation und/oder Sterilisation im Autoklaven sterilisiert wird. Alternativ hierzu ist es erfindungsgemäß auch möglich, die Blisterverpackung und Zubehörteile in separaten Sterilbeuteln zu verpacken und durch eine geeignete Ethylenoxidsterilisation und/oder geeignete α-, β- und/oder Röntgenstrahlensterilisation und/oder Sterilisation im Autoklaven zu sterilisieren.

Eine Gassterilisation kann alternativ oder zusätzlich zu der oben angegebenen Vorgehensweise (Einbringen der für den zweiten Sterilisationsschritt vorgesehenen Materialien in eine Sterilverpackung, Verschließen der Verpackung und anschließende Sterilisation durch ein in die Verpackung eindringendes Gas) auch dadurch erreicht werden, dass vor dem Verschließen der Verpackung das sterilisierende Gas zusammen mit den zu sterilisierenden Materialien in die Sterilverpackung eingebracht wird.

Nach einer bevorzugten Ausführungsform basiert das aushärtbare, medizinische Abformmaterial auf Polysiloxanen, alkoxysilylfunktionellen Polyethern, aziridinofunktionellen Polyethern oder acrylat- und/oder methacrylatfunktionellen Polyethern.

In einer besonders bevorzugten Ausführungsform handelt es sich bei dem Abformmaterial um ein Zwei-Komponenten-Abformmaterial, insbesondere um ein additionsvernetzendes Zwei-Komponenten-Silicon-Abformmaterial.

Erfindungsgemäß eignet sich für ein Verfahren der oben genannten Art insbesondere ein additionsvernetzendes Zwei-Komponenten-Silicon-Material als medizinisches Abformmaterial, welches in der ersten Komponente A
a) mindestens einen Hydrosilylierungskatalysator,
b) mindestens ein vinylendgestopptes Polydialkylsiloxan und
c) mindestens 0,001 bis insbesondere etwa 1 Gew.-% wenigstens eines Stabilisators, besonders bevorzugt 0,005 bis 0,5 Gew.-% wenigstens eines Stabilisators und ganz besonders bevorzugt 0,01 bis 0,1 Gew.-% wenigstens eines Stabilisators, insbesondere ein Divinyldisiloxan, Hydroxyanisol, Butylhydroxytoluol Divinylhexamethyltrisiloxan, Phenothiazine, Phosphite und/oder HALS (hindered amin ligth stabilisator)
   und in der zweiten Komponente B
d) mindestens ein Organohydrogenpolysiloxan
   enthält. Dabei sind der wenigstens eine Stabilisator und der wenigstens eine Hydrosilylierungskatalysator, bspw. ein Platin-Divinyldisiloxan-Komplex, insbesondere ein Platin-Divinyltetramethyldisiloxan-Komplex, derart aufeinander abgestimmt, dass bei einer Sterilisation mit dem oben genannten Verfahren keine nennenswerte Vorvernetzung eintritt, so dass sich die Aktivität des Hydrosilylierungskatalysators bzw. die Vernetzungsfähigkeit der Vinyl- und SiH-Gruppen im Wesentlichen nicht ändert, d.h. dass sich die Aushärtezeit nicht um ein Δ von mehr oder weniger als einer Minute verlängert oder verkürzt. Weiter beträgt die Änderung der Viskosität der Einzelkomponenten A und B weniger als etwa 20%, bevorzugt weniger als 10%, ganz besonders bevorzugt weniger als 5%. Die Komponenten, insbesondere der Katalysator, werden somit nicht durch die Sterilisation geschädigt bzw. durch Vorvernetzen unbrauchbar, so dass das erfindungsgemäße Abformmaterial ausreichend lange, d.h. wenigstens etwa 12 Monate, insbesondere wenigstens etwa 24 Monate, lagerstabil bleibt.
   Zusätzlich kann die Komponente A
e) mindestens ein nicht-funktionelles Polymer und/oder
f) Mineralöle und/oder Wachse und/oder
g) mindestens eine antibakteriell/antimikrobiell wirkende Substanz und/oder
h) mindestens eine röntgenopake Substanz und/oder
i) verstärkende und/oder nicht verstärkende Füllstoffe und/oder
j) Farbstoffe und/oder
k) Additive und/oder
l) Tenside
   und/oder die Komponente B
b) mindestens ein endgestopptes Vinylpolydialkylsiloxan und/oder
e) mindestens ein nicht-funktionelles Polymer und/oder
f) Mineralöle und/oder Wachse und/oder
g) mindestens eine antibakteriell/antimikrobiell wirkende Substanz und/oder
h) mindestens eine röntgenopake Substanz und/oder
i) verstärkende und/oder nicht verstärkende Füllstoffe und/oder
k) Additive und/oder
l) Tenside
   enthalten.

Nach einer bevorzugten Ausführungsform ist das nicht-funktionelle Polymer d) ein trimethylsiloxy-endgestopptes Polydialkylsiloxan und/oder die antibakteriell/antimikrobiell wirkende Substanz g) ausgewählt aus der Gruppe bestehend aus Mikrosilber, Nanosilber, (Mikro/Nano-)Kupferoxid, (Mikro/Nano-) Zinkoxid.

Ein weiterer Gegenstand der Erfindung ist ein Kit aus wenigstens einer Primärverpackung, die mit einer Komponente eines Mehrkomponentenabformmaterials befüllt ist und aus wenigstens einem Zubehörteil, insbesondere wenigstens einem dynamischen oder statischen Mischer, wenigstens einem Abformlöffel, wenigstens einem Spatel oder dgl. Mischzubehör, wenigstens einem Aktivierungskopf, wenigstens einem Dichtring, wenigstens einer Kartusche und/oder wenigstens einer Austragspistole oder dgl. Austragsgerät, wobei zumindest die Primärverpackung mit der Komponente nach einem erfindungsgemäßen Verfahren sterilisiert ist. Vorzugsweise ist das mindestens eine Zubehörteil ebenfalls sterilisiert.

Die Erfindung wird nachfolgend anhand von Ausführungsbeispielen und unter Bezugnahme auf die Zeichnung näher erläutert. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbeziehung.

Es zeigen schematisch:
- Fig. 1a - 1d: die Verfahrensschritte bei der erfindungsgemäßen Sterilisation eines Schlauchbeutels,
- Fig. 2a - 2c: die Verfahrensschritte bei der erfindungsgemäßen Sterilisation einer direkt befüllten Kartusche, und
- Fig. 3a - 3b: die Verfahrensschritte bei der erfindungsgemäßen Sterilisation einer Blisterverpackung.

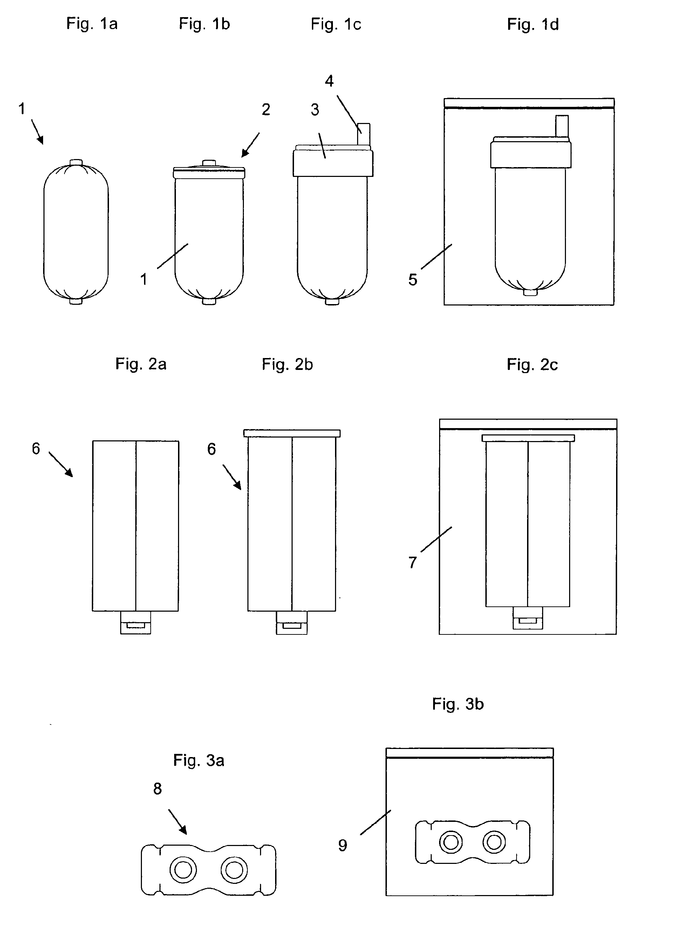

Nach der in den Figuren 1a bis 1d dargestellten Ausführungsform der Erfindung wird eine Komponente eines Ein- oder Mehrkomponentenabformmaterials in einen Schlauchbeutel 1 gefüllt, der einen Inhalt von etwa 1 ml bis bspw. etwa 400 ml aufweisen kann. In diesem Schlauchbeutel 1 wird die Komponente einer Sterilisation durch trockene Hitze bei einer Temperatur von etwa 138 °C bis etwa 150 °C unterzogen.

Wie in den Figuren 1b und 1c gezeigt, wird der sterilisierte Schlauchbeutel 1 dann zunächst mit einem Dichtring 2 bekappt, der bspw. auf den Schlauchbeutel 1 aufgeklebt ist. Weiter wird auf den Dichtring 2 ein kappenartiger Aktivierungskopf 3 aufgesetzt, der mit dem Dichtring 2 verrasten kann. Der Aktivierungskopf 3 ist mit einem Auslassstutzen 4 versehen, der mit einem Mischer oder dgl. zum Ausbringen der in dem Schlauchbeutel 1 aufgenommenen Komponente verbindbar ist.

Anschließend wird der Schlauchbeutel 1 mit Dichtring 2 und Aktivierungskopf 3 in einen in Figur 1d gezeigten Sterilbeutel 5 verpackt und versiegelt. Der Sterilbeutel 5 wird anschließend durch eine geeignete Ethylenoxidsterilisation und/oder geeignete α-, β- und/oder Röntgenstrahlensterilisation und/oder eine geeignete Sterilisation im Autoklaven sterilisiert.

Ebenso können in den Figuren nicht gezeigte Zubehörteile, wie z. B. dynamische oder statische Mischer und/oder wiederverwendbare Kartuschenkörper einzeln oder gemeinsam in Sterilbeutel verpackt und versiegelt werden und ebenfalls sterilisiert werden. Hierbei ist es auch möglich, die bekappten sterilisierten Schlauchbeutel und einzelne oder mehrere Zubehörteile in einen gemeinsamen Sterilbeutel zu verpacken, so dass diese gemeinsam einer Sterilisation unterworfen werden.

Die Komponenten können nach einer weiteren bevorzugten Ausführungsform der Erfindung in eine für die Sterilisation geeignete, direkt befüllbare Kunststoff-, Metall- oder metallbeschichtete Kartusche, insbesondere in eine in Figur 2a gezeigte Doppelkartusche 6, gefüllt werden. Die Doppelkartusche 6 weist auf der in Figur 2a unteren Seite verschlossene Auslassöffnungen auf, die mit einem nicht gezeigten Mischer verbindbar sind.

Wie in Figur 2b dargestellt, wird die Doppelkartusche 6 nach dem Befüllen auch auf der den Auslassöffnungen gegenüberliegenden Seite verschlossen und dann einer Sterilisation durch trockene Hitze unterworfen. Die derart sterilisierte Kartusche mit den Komponenten wird anschließend alleine oder zusammen mit Zubehörteilen, wie bspw. einem oder mehreren statischen Mischern und/oder einer Austragspistole, in einen in Figur 2c gezeigten Sterilbeutel 7 verpackt. Somit kann die Doppelkartusche 6 einzeln oder gemeinsam mit Zubehörteilen durch eine geeignete Ethylenoxidsterilisation und/oder geeignete α-, β- und/oder Röntgenstrahlensterilisation und/oder Sterilisation im Autoklaven sterilisiert werden.

Wenn die Komponenten in eine in Figur 3a dargestellte direkt befüllbare Blisterverpackung 8 als Primärverpackung eingefüllt sind, wird diese nach dem Verschließen einer Sterilisation durch trockene Hitze unterworfen. Die sterilisierte Blisterverpackung 8 mit den Komponenten wird ggf. zusammen mit nicht dargestelltem Mischzubehör, wie einem Spatel oder dgl., in einen in Figur 3b gezeigten Sterilbeutel 9 verpackt, der durch eine geeignete Ethylenoxidsterilisation und/oder geeignete α-, β- und/oder Röntgenstrahlensterilisation und/oder Sterilisation im Autoklaven sterilisiert wird.

## Patentansprüche

1. Verfahren zur Sterilisation von aushärtbaren, medizinischen Ein- oder Mehrkomponenten-Abformmaterialien, mit folgenden Schritten:
- Verpacken der Komponenten einzeln oder gemeinsam in wenigstens eine hitzebeständige Verpackung (1, 5, 6, 7, 8, 9), von denen zumindest eine Verpackung gasdicht ist, und
- anschließendes Sterilisieren der Komponenten durch vorzugsweise trockene Hitze bei einer Temperatur von über 138°C und bei Normaldruck.

2. Verfahren nach Anspruch 1, **dadurch gekennzeichnet, dass** vor dem gasdichten Verschließen der Verpackung (1, 5, 6, 7, 8, 9) Umgebungsluft vorzugsweise nahezu vollständig aus der Verpackung (1, 5, 6, 7, 8, 9) entfernt und/oder die Verpackung (1, 5, 6, 7, 8, 9) mit Inertgas befüllt wird.

3. Verfahren, insbesondere nach Anspruch 1 oder 2, **dadurch gekennzeichnet, dass** in einem ersten Schritt die nicht ausgehärteten Komponenten der Abformmaterialien jeweils in eine Primärverpackung (1, 6, 8) eingebracht werden, in einem zweiten Schritt die Primärverpackungen mit den darin enthaltenen Komponenten durch eine Hitzesterilisation sterilisiert werden, in einem dritten Schritt die sterilisierten Komponenten in den Primärverpackungen in eine Sekundärverpackung (5, 7, 9) eingebracht werden und in einem vierten Schritt diese Sekundärverpackung durch eine geeignete Gas-, Strahlen- und/oder Sterilisation in einem Autoklaven derart sterilisiert wird, dass sich die Aktivität und die Viskosität der Einzelkomponenten nicht ändert.

4. Verfahren nach einem der Ansprüche 1 bis 3, **dadurch gekennzeichnet, dass** die Primärverpackung (1, 6, 8) eine Kunststoff-Metall-Kunststoff-Verbundverpackung, insbesondere eine Folien- bzw. Blisterverpackung, eine Metall- oder Metallverbundtube, ein Metallverbundfolienschlauchbeutel, eine Metall- oder Metallverbundkartusche, insbesondere eine Polypropylen-Aluminium-Polypropylen-Verbundverpackung, Aluminiumtube, Zinntube, Aluminium-Zinn-Verbundtube, Aluminiumkartusche, Zinnkartusche oder Aluminium-Zinn-Verbundkartusche ist.

5. Verfahren gemäß einem der Ansprüche 1 bis 4, **dadurch gekennzeichnet, dass** die Primärverpackung (1, 6, 8) eine mit wenigstens einer Innenbeschichtung versehene Metallkartusche, -tube, -folie und/oder Blisterverpackung ist und/oder eine fluorierte Kunststoffprimärverpackung ist.

6. Verfahren gemäß einem der Ansprüche 1 bis 5, **dadurch gekennzeichnet, dass** die Hitzesterilisation bei einer Temperatur T größer als 138°C und kleiner oder gleich 160°C, bevorzugt bei einer Temperatur T größer oder gleich 140°C und kleiner oder gleich 150°C und besonders bevorzugt bei einer Temperatur T größer oder gleich 140°C und kleiner oder gleich 145°C durchgeführt wird.

7. Verfahren gemäß einem der Ansprüche 1 bis 6, **dadurch gekennzeichnet, dass** bei der Hitzesterilisation eine Behandlungszeit von größer oder gleich einer Stunde, bevorzugt größer oder gleich 1,5 Stunden und besonders bevorzugt größer oder gleich 2 Stunden gewählt wird.

8. Verfahren gemäß einem der Ansprüche 1 bis 7, **dadurch gekennzeichnet, dass** die Sekundärverpackung (5, 7, 9) ein Kunststoffbeutel ist.

9. Verfahren gemäß einem der Ansprüche 1 bis 8, **dadurch gekennzeichnet, dass** die Gassterilisation eine geeignete Ethylenoxidsterilisation und/oder die Strahlensterilisation eine geeignete α-, β-Strahlen- oder Röntgenstrahlensterilisation ist.

10. Verfahren gemäß einem der Ansprüche 1 bis 9, **dadurch gekennzeichnet, dass** bei der Gas- und/oder Strahlensterilisation und/oder der Sterilisation im Autoklaven eine Behandlungszeit von kleiner oder gleich einer Stunde, bevorzugt kleiner oder gleich 30 Minuten und besonders bevorzugt kleiner oder gleich 10 Minuten gewählt wird.

11. Verfahren gemäß einem der Ansprüche 1 bis 10, **dadurch gekennzeichnet, dass** wenigstens ein insbesondere zur Verarbeitung der Abformmaterialien zu verwendendes Zubehörteil mit in die Verpackung (1, 5, 6, 7, 8, 9) eingebracht und der Sterilisation unterzogen wird, wobei insbesondere wenigstens eine sterilisierte Primärverpackung (1, 6, 8) zusammen mit wenigstens einem insbesondere zur Verarbeitung der Abformmaterialien verwendeten Zubehörteil in eine gemeinsame Sekundärverpackung (5, 7, 9) eingebracht und/oder gemeinsam einer Sterilisation unterzogen werden.

12. Verfahren gemäß Anspruch 11, **dadurch gekennzeichnet, dass** das zur Verarbeitung der Abformmaterialien verwendete Zubehörteil wenigstens ein dynamischer oder statischer Mischer, wenigstens ein Abformlöffel, wenigstens ein Spatel oder dgl. Mischzubehör, wenigstens ein Aktivierungskopf, wenigstens ein Dichtring, wenigstens eine Kartusche und/oder wenigstens eine Austragspistole oder dgl. Austragsgerät ist.

13. Verfahren gemäß einem der Ansprüche 1 bis 12, **dadurch gekennzeichnet, dass** das Ein- oder Mehrkomponenten-Abformmaterial auf Polysiloxanen, alkoxyfunktionellen Polyethern, aziridinofunktionellen Polyethern oder acrylat- und/oder methacrylatfunktionellen Polyethern basiert.

14. Verfahren gemäß einem der Ansprüche 1 bis 13, **dadurch gekennzeichnet, dass** das Ein- oder Mehrkomponenten-Abformmaterial ein additionsvernetzendes Zwei-Komponenten-Silicon-Abformmaterial ist.

15. Verfahren nach einem der Ansprüche 3 bis 14 **dadurch gekennzeichnet, dass** in dem vierten Schritt eine Hitzesterilisation bei einer Temperatur T kleiner 138°C bei Normaldruck durchgeführt wird.

16. Zwei-Komponenten-Silicon-Abformmaterial mit zwei Komponenten A und B, enthaltend in der ersten Komponente A
a) mindestens einen Hydrosilylierungskatalysator,
b) mindestens ein vinylendgestopptes Polydialkylsiloxan und
c) mindestens 0,001 bis etwa 1 Gew.-%, bevorzugt 0,005 bis 0,5 Gew.-%, wenigstens eines Stabilisators, besonders bevorzugt 0,01 bis 0,1 Gew.-% wenigstens eines Stabilisators,
und in der zweiten Komponente B
d) mindestens ein Organohydrogenpolysiloxan,
**dadurch gekennzeichnet, dass** der wenigstens eine Stabilisator und der wenigstens eine Hydrosilylierungskatalysator derart aufeinander abgestimmt sind, dass sich die Aktivität des Hydrosilylierungskatalysators und/oder die Viskosität der Einzelkomponenten A und B bei einer Sterilisation mit einem Verfahren nach einem der vorhergehenden Ansprüche nicht ändert.

17. Zwei-Komponenten-Silicon-Abformmaterial gemäß Anspruch 16, **dadurch gekennzeichnet, dass** die Komponente A zusätzlich
e) mindestens ein nicht-funktionelles Polymer und/oder
f) Mineralöle und/oder Wachse und/oder
g) mindestens eine antibakteriell/antimikrobiell wirkende Substanz und/oder
h) mindestens eine röntgenopake Substanz und/oder
i) verstärkende und/oder nicht verstärkende Füllstoffe und/oder
j) Farbstoffe und/oder
k) Additive und/oder
l) Tenside
und/oder die Komponente B zusätzlich
b) mindestens ein endgestopptes Vinylpolydialkylsiloxan und/oder
e) mindestens ein nicht-funktionelles Polymer und/oder
f) Mineralöle und/oder Wachse und/oder
g) mindestens eine antibakteriell/antimikrobiell wirkende Substanz und/oder
h) mindestens eine röntgenopake Substanz und/oder
i) verstärkende und/oder nicht verstärkende Füllstoffe und/oder
k) Additive und/oder
l) Tenside
enthält.

18. Zwei-Komponenten-Silicon-Abformmaterial gemäß Anspruch 16 oder 17, **dadurch gekennzeichnet, dass** der wenigstens eine Stabilisator c) ein Divinyldisiloxan, Hydroxyanisol, Butylhydroxytoluol, Divinylhexamethyltrisiloxan, Phenothiazine, Phosphite und/oder HALS (hindered amin ligth stabilisator) ist, und/oder dass der Hydrosilylierungskatalysator a) ein Platin-Divinyldisiloxan-Komplex ist.

19. Zwei-Komponenten-Silicon-Abformmaterial gemäß einem der Ansprüche 16 bis 18, **dadurch gekennzeichnet, dass** die antibakteriell/antimikrobiell wirkende Substanz g) aus der Gruppe bestehend aus Mikrosilber, Nanosilber, (Mikro/Nano-)Kupferoxid, (Mikro/Nano-)Zinkoxid ausgewählt ist.

20. Kit aus wenigstens einer Primärverpackung, die mit wenigstens einer Komponente eines Mehrkomponenten-Abformmaterials befüllt ist, und aus wenigstens einem Zubehörteil, insbesondere wenigstens einem dynamischen oder statischen Mischer, wenigstens einem Abformlöffel, wenigstens einem Spatel oder dgl. Mischzubehör, wenigstens einem Aktivierungskopf, wenigstens einem Dichtring, wenigstens einer Kartusche und/oder wenigstens einer Austragspistole oder dgl. Austragsgerät, **dadurch gekennzeichnet, dass** zumindest die Primärverpackung (1, 6, 8) mit der wenigstens einen Komponente nach einem Verfahren gemäß einem der Ansprüche 1 bis 15 sterilisiert ist.

21. Kit gemäß Anspruch 20, **dadurch gekennzeichnet, dass** das wenigstens eine Zubehörteil ebenfalls sterilisiert ist.
